# EUROPÄISCHE PATENTANMELDUNG

(11) **EP 0 945 517 A2**
(43) Veröffentlichungstag der Anmeldung: **29.09.1999**
(21) Anmeldenummer: 99101849.0
(22) Anmeldetag: 28.01.1999
(51) Int. Cl.: C12P 13/20, C07K 5/06

(54) **Verfahren zur Herstellung von Z-L-Asparaginsäure-Dinatriumsalz**

(30) Priorität: 02.02.1998 AT 16398
(71) Anmelder: DSM Fine Chemicals Austria GmbH, 4021 Linz (AT)
(72) Erfinder: Giselbrecht, Karl-Heinz, 4061 Pasching (AT); Raml, Walter, 4202 Hellmonsödt (AT)
(74) Vertreter: Klostermann, Ingrid

(57) **Zusammenfassung**

Verfahren zur Herstellung von Z-L-Asparaginsäure-Dinatriumsalz aus Fumarsäure, bei welchem
a) Fumarsäure in einem inerten Verdünnungsmittel in Gegenwart von Aspartase oder Aspartase-produzierenden Mikroorganismen mit einem Überschuß an Ammoniak zu Ammonium-L-Aspartat umgesetzt, anschließend
b) die Ammonium-L-Aspartat hältige Lösung mit Natriumhydroxid versetzt wird, wobei sich je nach eingesetzter Menge an Natriumhydroxid das Mono- oder Dinatrium-L-Aspartat oder ein Gemisch davon bildet und
c) der sich abspaltende und überschüssiger Ammoniak bei Normaldruck und einer Rückflußtemperatur von 90 - 120°C unter Verweilzeiten von 0,2 bis 2 Minuten kontinuierlich destillativ entfernt und in eine Fumarsäuresuspension, die als Ausgangslösung für weitere enzymatische Umsetzungen verwendet wird, rückgeführt wird, worauf
d) die verbleibende Natrium-L-Aspartat hältige Lösung mit Benzyloxycarbonylchlorid bei einem pH-Wert zwischen 9 und 14 bei gleichzeitiger Zugabe von Natriumhydroxid reagieren gelassen wird, sodaß sich Z-L-Asparaginsäure-Dinatriumsalz bildet.

## Beschreibung

L-Asparaginsäure ist ein essentieller Ausgangsstoff für die Synthese des Dipeptides Aspartam, ein künstlicher Süßstoff mit einer +/- 200-fachen Süßkraft von Saccharose.

Für die Synthese von Aspartam wurden bereits eine Vielzahl von chemischen und enzymatischen Verfahren beschrieben, bei welchen vor allem L-Asparaginsäure und Phenylalaninmethylester auf unterschiedlichste Weisen, etwa mit oder ohne enzymatischer Katalyse, miteinander gekoppelt werden. Dabei wird beispielsweise von fester Asparaginsäure ausgegangen, die nach Umwandlung in das Dinatriumsalz mit Benzyloxycarbonylchlorid (Z-Cl) in wäßriger Lösung zu Z-L-Asparaginsäure-Dinatriumsalz umgesetzt wird. Asparaginsäure wird dabei, wie beispielsweise in EP-A-0 127 940 beschrieben, aus Maleinsäure, die zur Fumarsäure isomerisiert wird, auf enzymatischem Wege über das Ammonium-L-Aspartat und anschließender Kristallisation in Gegenwart einer Säure hergestellt. Dabei fallen in der Mutterlauge äquimolare Mengen an Ammoniumsalzen an, die entsprechend entsorgt werden müssen. Bei diesem Verfahren ist außerdem eine Vielzahl von Schritten erforderlich, um Z-L-Asparaginsäure-Dinatriumsalz zu erhalten. Weiters ist zu beachten, daß eine Lösung von Z-L-Asparaginsäure-Dinatriumsalz einer strengen Spezifikation bezüglich Rest-Ammoniakgehalt, Rest-Fumarsäuregehalt, Farbe und optische Reinheit unterliegt.

Aufgabe der vorliegenden Erfindung war es daher, ein Verfahren zu finden, bei welchem die Ammoniumionen-hältigen Abwasser vermieden, die Anzahl der Schritte zum Z-L-Asparaginsäure-Dinatriumsalz reduziert und die erforderlichen Spezifikationen erfüllt werden.

Unerwarteterweise konnte diese Aufgabe durch ein Verfahren gelöst werden, bei welchem, ausgehend von Fumarsäure, Ammonium-L-Aspartat direkt in das Dinatriumsalz der L-Asparaginsäure bei gleichzeitiger quantitativer Rückführung des Ammoniaks überführt wird.

Gegenstand der vorliegenden Erfindung ist demnach ein Verfahren zur Herstellung von Z-L-Asparaginsäure-Dinatriumsalz aus Fumarsäure, das dadurch gekennzeichnet ist, daß
a) Fumarsäure in einem inerten Verdünnungsmittel in Gegenwart von Aspartase oder Aspartase-produzierenden Mikroorganismen mit einem Überschuß an Ammoniak zu Ammonium-L-Aspartat umgesetzt, anschließend
b) die Ammonium-L-Aspartat hältige Lösung mit Natriumhydroxid versetzt wird, wobei sich je nach eingesetzter Menge an Natriumhydroxid das Mono- oder Dinatrium-L-Aspartat oder ein Gemisch davon bildet und
c) der sich abspaltende und überschüssiger Ammoniak bei Normaldruck und bei einer Rückflußtemperatur von 90 bis 120°C unter Verweilzeiten von 0,2 - 2 Minuten kontinuierlich destillativ entfernt und in eine Fumarsäuresuspension, die als Ausgangslösung für weitere enzymatische Umsetzungen verwendet wird, rückgeführt wird, worauf
d) die verbleibende Natrium-L-Aspartat hältige Lösung mit Benzyloxycarbonylchlorid bei einem pH-Wert zwischen 9 und 14 bei gleichzeitiger Zugabe von Natriumhydroxid reagieren gelassen wird, sodaß sich Z-L-Asparaginsäure-Dinatriumsalz bildet.

Im ersten Schritt a) des erfindungsgemäßen Verfahrens wird Fumarsäure in einer enzymatischen Reaktion zu Ammonium-L-Aspartat umgesetzt. Das Ausgangsprodukt für das erfindungsgemäße Verfahren, die Fumarsäure kann beispielsweise durch Isomerisierung von Maleinsäure hergestellt werden.

Für die enzymatische Reaktion wird Fumarsäure bis zur Löslichkeitsgrenze in ein inertes Verdünnungsmittel eingerührt, sodaß eine Suspension erhalten wird. Als inerte Verdünnungsmittel eignen sich Wasser, Wasser/Ethanol- oder Wasser/Aceton Gemische und dergleichen.
Bevorzugt wird Wasser eingesetzt. In die Fumarsäure-Suspension wird sodann bei Raumtemperatur Ammoniak verflüssigt oder in Form einer 10 bis 35 Gew.%igen Lösung eingeleitet, wodurch sich die Temperatur bis zu 60°C erhöht und ein pH-Wert zwischen 8 und 9 einstellt.
Bevorzugt wird eine wäßrige 20 bis 30 Gew.%ige Ammoniaklösung verwendet. Ammoniak wird dabei im Überschuß, bevorzugt in 1 - 30 %igem Überschuß, zugesetzt.
In das so erhaltene System, vorzugsweise eine Lösung, wird sodann bei 20 bis 60°C, bevorzugt bei 30 bis 50°C das Enzym Aspartase oder ein Aspartaseproduzierender Mikroorganismus, eingerührt. Bei dieser Zugabe an Enzym- bzw. Aspartase-produzierenden Mikroorganismus ist es von Vorteil, wenn durch die Ammoniakzugabe eine Lösung erhalten wird, da im Falle einer Suspension durch Adsorption des Enzyms und dadurch bedingten Aktivitätsverlust mehr Enzym erforderlich ist. Für einen fast quantitativen Umsatz nach bis zu 24 bis 30 Stunden sind dabei 30 bis 50 IU (Enzymaktivität) pro Mol Fumarsäure erforderlich. Aspartase-produzierende Mikroorganismen sind beispielsweise Pseudomonas fluorescens, Protens vulgaris, Pseudomonas aeruginosa, Serratia marcescens, Bacterium succinium, Bacillus subtilis, Aerobacter aerogenes, Micrococcus sp. Escherichia coli u. a.
Weitere geeignete Aspartase-produzierende Mikroorganismen sind beispielsweise in US 3,791,926 und US 3,198,712 beschrieben.
Bei dem erfindungsgemäßen Verfahren kann weiters gereinigte oder synthetische Aspartase eingesetzt werden. Das Enzym bzw. der Aspartase-produzierende Mikroorganismus kann in flüssiger oder in immobilisierter Form wie beispielsweise in EP 0 127 940 beschrieben, zugesetzt werden.
Nach vollendeter Reaktion, das Reaktionsende kann beispielsweise photometrisch ermittelt werden, wird die Ammonium-L-Aspartat haltige Lösung gegebenenfalls auf 20 bis 30°C abgekühlt und im 2. Schritt (b) mit Natriumhydroxid versetzt. Die Menge an zugesetztem Natriumhydroxid ist dabei abhängig vom gewünschten Natriumsalz, bzw. vom gewünschten pH-Wert der Produktlösung. Für die Weiterreaktion eignen sich dabei sowohl das Mononatrium- und das Dinatriumsalz als auch Gemische davon. Natriumhydroxid kann dabei in fester Form, oder als Natronlauge eingesetzt werden. Bevorzugt wird Natronlauge eingesetzt. Die Reaktionslösung erwärmt sich dabei um etwa 10 - 30°C.

Erfindungsgemäß wird Ammoniak kontinuierlich aus dem Reaktionsgemisch bevorzugt unter Verwendung eines Dünnschichtverdampfers, Fallfilmverdampfer oder eines Kurzwegverdampfers destillativ entfernt. Die Reaktions- bzw. Rückflußtemperatur beträgt dabei 90 - 120°C, bevorzugt 100 - 110°C.

Die Destillation wird unter Normaldruck und unter sehr kurzen Verweilzeiten von etwa 0,2 bis 2 Minuten, bevorzugt von etwa 0,5 bis 1,5 Minute durchgeführt. Der abdestillierte Ammoniak wird sodann in eine Fumarsäuresuspension, die nach weiterer Zugabe von Ammoniak wieder als Ausgangslösung für die nächste enzymatische Umsetzung verwendet werden kann, rückgeführt. (Schritt c) Gemäß dieser Verfahrensweise bei der Abtrennung des Ammoniaks wird eine praktisch quantitative Entfernung des Ammoniaks (Restgehalt < 100 ppm) gewährleistet und Razemisierungsreaktionen (D-Asp < 0,1 Gew.%) sowie eine Gelbfärbung der verbleibenden Lösung und Rückspaltung zu Fumarsäure unterdrückt bzw. praktisch vollständig verhindert.

Die so erhaltene Natrium-L-Aspartat-Lösung wird sodann im nächsten Schritt d) bei einer Temperatur von 30 bis 60°C mit Benzyloxycarbonylchlorid (Z-Cl) versetzt, wobei durch gleichzeitiges Zutropfen von Natronlauge ein pH-Wert zwischen 9 und 14 bevorzugt zwischen 11 und 12 gehalten wird. Z-Cl kann dabei in einer äquimolaren Menge, aber auch in leichtem Überschuß zugesetzt werden. Nach der Z-Cl Zugabe wird das Reaktionsgemisch noch 0,5 bis 4 Stunden bei der Reaktionstemperatur stehen gelassen und anschließend auf Raumtemperatur abgekühlt, worauf es zu einer Phasentrennung kommt.

Die wäßrige Phase wird sodann gewaschen, beispielsweise mit Methyl-tert-butylether, Diisobutylether, Toluol oder anderen mit Wasser nicht-mischbaren Lösungsmitteln.
Die organische Phase wird verworfen und Z-L-Asparaginsäure als Dinatriumsalz in wäßriger Lösung erhalten. Zur Entfernung von eventuellen Restmengen an organischen Lösungsmitteln in der wäßrigen Phase, wird bei Raumtemperatur ein Vakuum angelegt.
Durch das erfindungsgemäße Verfahren wird Z-L-Asparaginsäure-Dinatriumsalz als klare, leicht gelbliche Lösung erhalten, die bei Bedarf für die Weiterverarbeitung zu Aspartam, mittels Kopplung mit Phenylalanin verwendet werden kann. Durch das erfindungsgemäße Verfahren werden Ammoniumionen-hältige Abwässer vermieden und Ammoniak quantitativ für weitere enzymatische Umsetzungen von Fumarsäure rückgeführt.

Weiters ist es bei dem erfindungsgemäßen Verfahren nicht erforderlich, L-Asparaginsäure oder eines der Zwischenprodukte zu isolieren.

Die so erhaltene Dinatriumsalzlösung von Z-L-Asparaginsäure entspricht allen Spezifikationen bezüglich Restgehalt von Fumarsäure (< 0,3 Gew.%) und Ammoniak (< 100 ppm), Farbe (Farbzahl APHA < 25) und optischer Reinheit (D-Asp. < 0,1 Gew.%).

### Beispiel 1

### a) Enzymatische Umsetzung

In einem 5 l Doppelmantelgefäß wurden 2070 ml Trinkwasser vorgelegt und 973 g Fumarsäure (8,38 Mol) eingerührt. In die Fumarsäuresuspension wurden anschließend 1450 ml 25 Gew.%ige Ammoniak-Lösung (1314 g) während 30 Minuten eingebracht. Dabei wurde eine Reaktionstemperatur von 45 - 50°C und ein pH-Wert von 8,5 erreicht. Bei 45°C und unter leichtem Rühren wurden in die klare Lösung 1,4 ml Aspartase Lösung (245 IU/ml) eingebracht. Danach wurde die Rührung ausgeschaltet und die Reaktionslösung bei 45°C gehalten. Der Reaktionsverlauf wurde photometrisch verfolgt. Eine nahezu quantitative Umsetzung (> 99 %) wurde nach 25,5 Stunden erreicht.

### b) und c) Austreiben von Ammoniak

In 2445 ml einer gemäß a) erhaltenen Lösung von NH₄-L-Asparaginsäure wurde nach Abkühlen auf 25°C 842,5 g 50 Gew.%ige Natronlauge innerhalb von 15 Minuten eingebracht. Dabei erwärmte sich die Lösung auf 50°C. Es wurde ein pH-Wert von 11,5 gemessen. Anschießend wurde der sich abspaltende und überschüssige Ammoniak mittels eines Dünnschichtverdampfers mit Membran-Dosierpumpe bei Normaldruck aus der Na-Salz-L-Asparaginsäurelösung entfernt.

Die Temperatur des Ölbades betrug 175°C, die Rotordrehzahl lag bei 24% (= 300 U/Min).

| Zeit | ml in Vorlage | Sumpftemperatur (°C) | ml Kopf | ml Sumpf |
|---|---|---|---|---|
| 0 h | 1000 | 88,2 | | |
| nach 40 min | 500 | 94,3 | 230 | 270 |
| 1 h 23 min | 0 | 82,3 | 480 | 510 |

Es wurde eine Dinatrium-L-Aspartat-Lösung mit einem Gehalt von 33,5 Gew.% an L-Asparaginsäure erhalten. Dabei konnte keine Racemisierung beobachtet werden. Der Gehalt an D-Asparaginsäure betrug 0,04 Gew.% (D-Asp: L-Asp = < 0,1 : > 99,9 %). Der abdestillierte Ammoniak wurde in eine Fumarsäuresuspension, die als Ausgangslösung für eine weitere Reaktion diente, eingeleitet. Der Restammoniakgehalt lag bei 20 ppm.
Der Gehalt an Fumarsäure betrug < 0,04 Gew.%, Äpfelsäure und Asparagin konnte nicht nachgewiesen werden.

### d) Herstellung von Dinatrium Z-(L)-asDartat

Es wurden 80 ml einer analog Beispiel 1a - c) hergestellten Dinatrium L-Aspartat Lösung mit 18,1 Gew.% an L-Asparaginsäure (0,11 mol) auf 45°C erwärmt. Unter Rühren wurden 20,5 g Benzyloxycarbonylchlorid (Z-Cl) (Gehalt: 92,5 nach GC) (0,11 Mol) während 1 Stunde zugetropft. Gleichzeitig wurde durch Zutropfen von 50 Gew.%ige Natronlauge der pH-Wert zwischen 10,9 und 13,8 gehalten. Insgesamt wurden dabei 7,8 g 50 Gew.%ige Natronlauge zugegeben. Nach dem Zutropfen wurde die Reaktionslösung für 2,5 Stunden bei 45-47 °C gehalten. Der pH-Wert betrug konstant 12,1. Nach dem Abkühlen auf 25°C wurde die wässrige Lösung 2 mal mit jeweils 92 ml Methyl-tert- butylether behandelt. Die organische Phase wurde verworfen; die wässrige Phase wurde 30 Minuten bei 20°C und 20 mbar im Rotavapor von Lösungsmittelresten befreit.
Es wurden 101 g einer leicht gelblichen, klaren Lösung erhalten. Der Gehalt betrug 30,8 Gew.% Dinatrium-Z-(L)-Aspartat.

### Beispiel 2

### Herstellung von Mononatrium-L-Aspartatlösung

Analog Beispiel 1 b) und c) wurde in 1300 ml einer gemäß 1a) erhaltenen Lösung nach Abkühlen auf 25°C 588 g 20 Gew.%ige Natronlauge innerhalb von 15 Minuten eingebracht. Dabei erwärmte sich die Lösung auf 50°. Bei Normaldruck wurde die Gesamtmenge des Ammoniaks analog Beispiel 1c destillativ entfernt und die Reaktionslösung auf 1200 ml eingeengt. Restammoniakgehalt < 100 ppm.

Es wurde eine Mononatrium-L-Aspartat-Lösung mit einem Gehalt von 25,9 Gew.% an L-Asparaginsäure erhalten. Dabei konnte keine Racemisierung beobachtet werden.
Der Gehalt an Fumarsäure betrug < 0,1 Gew.%, Äpfelsäure und Asparagin konnte nicht nachgewiesen werden.

Die Lösung wurde analog Bsp. 1d zu Dinatrium Z-(L)-aspartat weiterverarteitet.

### Beispiel 3

### Herstellung von Dinatrium Z-(L)-Aspartat mit einem Überschuß von Z-Cl

Es wurden 64,2 ml einer Dinatrium-L-Aspartat Lösung mit 18,1 Gew.% L-Asparaginsäure (0,09 Mol), hergestellt analog Beispiel 1 a - c, auf 45°C erwärmt. Unter Rühren wurden 17,7 g Benzyloxycarbonylchlorid (Z-Cl, Gehalt: 92,5 nach GC) (0,10 Mol) während 1 Stunde zugetropft. Gleichzeitig wurde durch Zutropfen von 50 Gew.%ige Natronlauge der pH Wert zwischen 9,4 und 11,8 gehalten. Insgesamt wurden dabei 8,8 g 50 Gew.%ige Natronlauge zugegeben. Nach dem Zutropfen wurde die Reaktionslösung für 2,5 Stunden bei 45°C gehalten. Der pH-Wert betrug konstant 11,3. Nach dem Abkühlen auf 25°C wurde die wässrige Lösung 2 mal mit jeweils 46 ml Methyl-tert-butylether behandelt. Die organische Phase wurde verworfen; die wässrige Phase wurde 30 Minuten bei 20°C und 20 mbar im Rotavapor von Lösungsmittelresten befreit.

Es wurden 84,2 g einer leicht gelblichen, klaren Lösung erhalten. Der Gehalt betrug 31,4 Gew.% Dinatrium-Z-(L)-Aspartat.

## Patentansprüche

1. Verfahren zur Herstellung von Z-L-Asparaginsäure-Dinatriumsalz aus Fumarsäure, dadurch gekennzeichnet, daß
a) Fumarsäure in einem inerten Verdünnungsmittel in Gegenwart von Aspartase oder Aspartase-produzierenden Mikroorganismen mit einem Überschuß an Ammoniak zu Ammonium-L-Aspartat umgesetzt, anschließend
b) die Ammonium-L-Aspartat hältige Lösung mit Natriumhydroxid versetzt wird, wobei sich je nach eingesetzter Menge an Natriumhydroxid das Mono- oder Dinatrium-L-Aspartat oder ein Gemisch davon bildet und
c) der sich abspaltende und überschüssiger Ammoniak bei Normaldruck und einer Rückflußtemperatur von 90 - 120°C unter Verweilzeiten von 0,2 bis 2 Minuten kontinuierlich destillativ entfernt und in eine Fumarsäuresuspension, die als Ausgangslösung für weitere enzymatische Umsetzungen verwendet wird, rückgeführt wird, worauf
d) die verbleibende Natrium-L-Aspartat hältige Lösung mit Benzyloxycarbonylchlorid bei einem pH-Wert zwischen 9 und 14 bei gleichzeitiger Zugabe von Natriumhydroxid reagieren gelassen wird, sodaß sich Z-L-Asparaginsäure-Dinatriumsalz bildet.

2. Verfahren nach Anspruch 1, dadurch gekennzeichnet, daß als inertes Verdünnungsmittel Wasser, Wasser/Ethanol oder Wasser/Aceton Gemische verwendet werden.

3. Verfahren nach Anspruch 1, dadurch gekennzeichnet, daß Fumarsäure in Gegenwart von 30 bis 50 IU (Enzymaktivität) pro Mol Fumarsäure umgesetzt wird.

4. Verfahren nach Anspruch 1, dadurch gekennzeichnet, daß Ammoniak verflüssigt oder in Form einer wäßrigen 10 bis 35 Gew.%igen Lösung eingesetzt wird.

5. Verfahren nach Anspruch 1, dadurch gekennzeichnet, daß Ammoniak in einem 1 - 30 %igen Überschuß eingesetzt wird.

6. Verfahren nach Anspruch 1, dadurch gekennzeichnet, daß der Ammoniak mittels Dünnschichtverdampfer, Fallfilmverdampfer oder Kurzwegverdampfer entfernt wird.

7. Verfahren nach Anspruch 1, dadurch gekennzeichnet, daß der Ammoniak bei einer Rückflußtemperatur von 100 - 110 °C entfernt wird.

8. Verfahren nach Anspruch 1, dadurch gekennzeichnet, daß die Natrium-L-Aspartat hältige Lösung bei 30 bis 60°C mit Benzyloxycarbonylchlorid versetzt wird.

9. Verfahren nach Anspruch 1, dadurch gekennzeichnet, daß im Anschluß an die Reaktion von Natrium-L-Aspartat und Benzyloxycarbonylchlorid, nach erfolgter Phasentrennung, die wäßrige Phase mit einem mit Wasser nicht mischbaren Lösungsmittel gewaschen wird.

10. Verfahren nach Anspruch 9, dadurch gekennzeichnet, daß die das Z-L-Asparaginsäure-Dinatriumsalz enthaltende wäßrige Phase zur Weiterverarbeitung zu Aspartam mittels Kopplung mit Phenylalanin verwendet wird.
